# FASCICULE DE BREVET EUROPEEN

(11) **EP 0 836 862 B1**
(45) Date de publication et mention de la délivrance du brevet: **17.03.1999**
(21) Numéro de dépôt: 97402300.4
(22) Date de dépôt: 01.10.1997
(51) Int. Cl.: A61M 35/00, A45D 34/04

(54) **Ensemble de conditionnement et d'application**
Verpackungseinheit und Abgabevorrichtung
Storage case and applicator assembly

(30) Priorité: 15.10.1996 FR 9612565
(43) Date de publication de la demande: 22.04.1998
(73) Titulaire: L'OREAL, 75008 Paris (FR)
(72) Inventeur: Gueret, Jean-Louis H., 75018 Paris (FR)
(74) Mandataire: Boulard, Denis

(56) Documents cités:
- EP-A- 0 380 182
- EP-A- 0 612 488
- EP-A- 0 667 301
- EP-A- 0 688 516
- DE-A- 4 434 793
- FR-A- 2 290 863

## Description

La présente invention a trait à un ensemble de conditionnement et d'application pouvant être utilisé notamment dans les domaines de la pharmacie, de la cosmétique, de la dermatologie, des soins capillaires, etc. L'invention concerne de manière plus particulière un dispositif du type comportant un applicateur solide ou semi solide que l'on doit mettre en contact avec une composition liquide avant application et apte à se solubiliser ou se gélifier en surface au contact d'une composition liquide. A titre d'exemple, il peut s'agir d'un raisin de rouge à lèvres faiblement délitable et dont la surface doit être mise en contact, avec de l'eau par exemple, afin de le ramollir en surface et de favoriser ainsi le délitage du rouge à lèvres. A titre d'exemple encore, l'applicateur peut être de type " pierre d'alun" utilisé pour la cautérisation de plaies. Dans cette dernière application, l'applicateur contient des substances actives hydrosolubles tels que des sulfates d'aluminium et d'ammonium mixtes qui se solubilisent en présence d'une composition liquide telle que de l'eau. L'invention s'applique également pour l'application d'un produit pouvant avoir un effet cosmétique et un effet traitant, l'effet cosmétique étant produit par l'applicateur lui même, l'effet traitant étant obtenu par une solution liquide au contact de laquelle est mis l'applicateur. A titre d'exemple, il s'agit d'un raisin de rouge à lèvres que l'on met au contact d'une composition traitante, de type composition anti gerçures. Il peut s'agir également d'un savon de stéarate d'ammonium ou de potassium, ou d'un stick tenseur, par exemple d'alcool polyvinylique (PVA) apte à effacer les rides. Il peut s'agir encore d'un composé, tel qu'un émollient, qui ne peut pas être introduit dans le raisin lors de sa fabrication.

Le brevet Européen 612 488 décrit un applicateur comprenant un réservoir contenant un produit à appliquer, un capuchon destiné à fermer le réservoir et un porte applicateur supportant un élément déformable d'application du produit, en mousse ou en élastomère basse dureté. L'élément déformable porte en surface des aspérités et est doté d'une grande mémoire de forme. Le réservoir de produit est limité par un embout capillaire en forme de doigt de gant ayant un fond muni d'un siège percé d'au moins un orifice capillaire contre lequel l'élément déformable est appliqué et déformé dans la position de fermeture du réservoir par le capuchon.

C'est un objet de l'invention que de réaliser un dispositif de conditionnement et d'application dont au moins une partie de la surface de l'applicateur est maintenue au contact d'une composition liquide.

Selon l'invention, on réalise un ensemble de conditionnement et d'application d'axe principal X, comportant un applicateur et un réservoir contenant une composition liquide au contact de laquelle doit être mis l'applicateur avant application, caractérisé en ce que l'applicateur contient un produit apte à se solubiliser ou se gélifier en surface au contact de la dite composition liquide, l'applicateur étant mis en contact avec la composition liquide au travers d'un élément élastiquement déformable apte à contenir une quantité de ladite composition liquide, et pouvant passer sélectivement d'une position comprimée dans laquelle au moins une partie de la surface de l'applicateur est en appui, selon l'axe X, sur une surface de restitution de l'élément déformable de manière à être en contact avec la composition liquide, à une position de repos, le passage de la position comprimée à la position de repos provoquant le chargement de l'élément déformable en composition liquide par pompage au travers d'une surface de chargement de l'élément déformable.

Avantageusement, l'élément élastiquement déformable est en mousse ou en élastomère basse dureté, et est porté par le réservoir, un capuchon étant monté de manière amovible sur le réservoir et portant un porte applicateur sur lequel est monté l'applicateur, ledit applicateur étant, en position fermée du capuchon en appui sur la surface de restitution de l'élément déformable de manière à comprimer ledit élément déformable selon l'axe X et à maintenir l'applicateur au contact avec la composition liquide, l'ouverture du capuchon et la sortie de l'applicateur provoquant par effet de pompage le chargement de l'élément déformable au travers de la surface de chargement. Le réservoir peut être constitué d'un tube. Il peut être, au moins en partie, constitué d'un matériau translucide de manière à permettre de visualiser la quantité de liquide restant dans le réservoir.

La disposition de l'élément déformable selon l'invention permet la réalisation, de façon simple, de plusieurs fonctions. L'élément déformable, comprimé sur le siège assure l'étanchéité du réservoir. La mémoire de forme de l'élément déformable permet la fonction de pompage et d'aspiration du produit lors de l'ouverture et du retrait de l'applicateur, permettant ensuite la mise en contact de l'applicateur avec la composition liquide en position fermée du capuchon. Il permet en outre de rattraper le jeu dû, le cas échéant, à l'usure de l'applicateur (dans le cas d'un rouge à lèvres par exemple). L'élément déformable peut présenter un rôle abrasif pour favoriser par exemple, le délitage à l'eau de l'applicateur.

L'élément déformable peut être monté à l'intérieur d'un organe de type doigt de gant comportant un fond au contact duquel est placé la surface de chargement, ledit fond présentant des moyens aptes à retenir par effet de tension superficielle ou capillarité une quantité de ladite composition liquide. De tels moyens peuvent comporter au moins un orifice et/ou au moins une fente, ménagés dans le fond du doigt de gant.

Avantageusement, le fond du doigt de gant comporte une pluralité de fentes formant des portions concentriques d'arcs de cercles. Selon une alternative, le fond du doigt de gant comporte une pluralité de fentes radiales. De telles fentes et/ou orifices peuvent déboucher dans le réservoir selon une portion évasée, tronconique, et faisant office de réservoir par effet de tension superficielle ou par effet de capillarité.

Selon un mode de réalisation particulier, le fond comporte un orifice central, pouvant être obturé sélectivement par une valve dont l'ouverture est provoquée par la compression de l'élément déformable, l'ouverture du capuchon et la sortie de l'applicateur provoquant la fermeture de la valve. Alternativement, l'ouverture de la valve est provoquée par l'ouverture du capuchon et la sortie de l'applicateur, la fermeture est provoquée par la compression de l'élément déformable.

L'applicateur peut se présenter sous la forme d'un raisin d'un produit solide ou semi solide, délitable en surface au contact d'une composition liquide, et est monté sur le porte applicateur par l'intermédiaire d'un mécanisme formant ressort de manière à compenser le raccourcissement de l'applicateur au fil des utilisations. Alternativement, le raccourcissement de l'applicateur est compensé en montant l'élément déformable dans le fond du doigt de gant par l'intermédiaire d'un mécanisme formant ressort.

L'élément déformable peut être monté dans le fond du doigt de gant par collage, soudure, bouterollage, ou par claquage, par l'intermédiaire d'une collerette annulaire.

La surface de restitution de l'élément déformable peut être recouverte d'un revêtement de flocage, ou d'un film perforé d'élastomère thermoplastique, de plastique, ou d'une couche de feutre, ou d'un textile. Cette caractéristique permet entre autres, d'ajuster la quantité de liquide transférée sur l'applicateur.

Selon l'invention l'applicateur peut être une pierre d'alun, un raisin de rouge à lèvres, d'un stick d'alcool polyvinylique (PVA), de gomme de guar, ou un stick de produit de traitement cutané. A titre d'exemple, la composition liquide contient de l'eau, ou tout autre solvant, et /ou des conservateurs, et/ou des émollients et/ou des composés actifs, la composition liquide devant être apte à être pompée par l'élément déformable.

Pour mieux faire comprendre l'objet de l'invention, on va en décrire maintenant, à titre d'exemples purement illustratifs et non limitatifs, des modes de réalisation préférés, représentés sur les dessins annexés. Parmi ces dessins:
- la figure 1 est une vue en coupe partielle d'un ensemble d'application selon l'invention;
- la figure 2 est analogue à la figure 1, mais l'ensemble d'application y est représenté en position ouverte;
- la figure 3 est une vue en coupe partielle d'une variante d'ensemble d'application selon l'invention;
- la figure 4 représente une vue en coupe d'un agencement particulier de l'élément sur lequel est monté l'élément déformable;
- la figure 5 est une vue en coupe partielle d'une variante d'ensemble d'application selon l'invention;
- les figures 6/I à 6/V montrent différentes formes de section transversale d'orifice de retenue de liquide par effet de tension superficielle;
- les figures 7 et 8 représentent en coupe longitudinale deux variantes d'orifice de retenue de liquide par effet de tension superficielle;
- la figure 9 illustre une variante de montage de l'élément déformable sur l'ensemble d'application; et
- les figures 10a et 10b illustrent une autre variante de montage de l'élément déformable sur l'ensemble d'application.

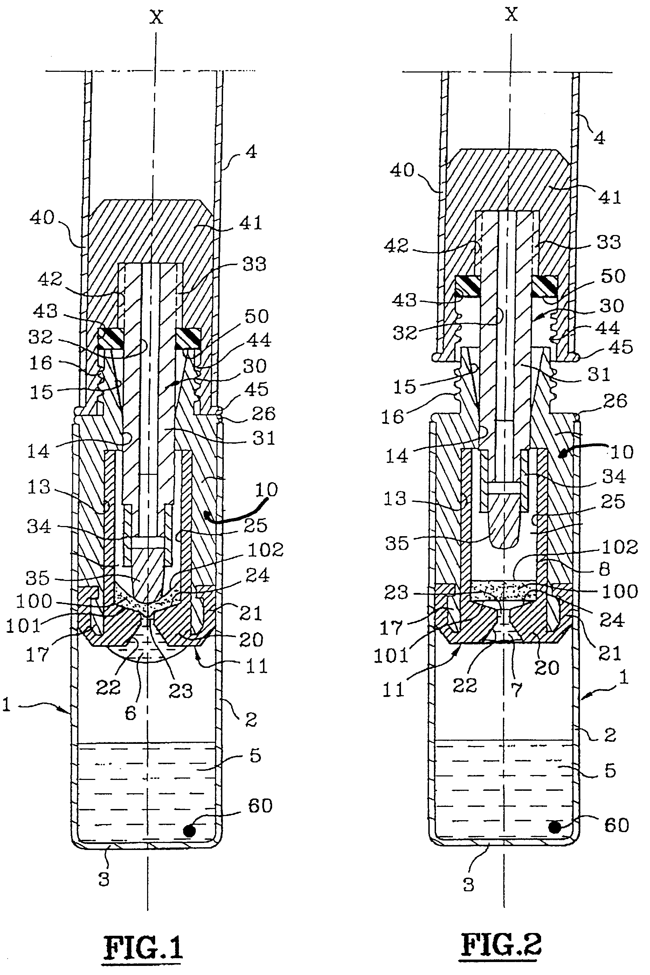

Selon la figure 1, l'ensemble d'application 1 comprend un réservoir 2 de forme cylindrique, avec un fond 3 et ouvert à une extrémité. Par cette extrémité ouverte, est emmanchée à force une entretoise 10, de diamètre légèrement inférieur au diamètre interne du réservoir 2, jusqu'à venue en butée d'une collerette 26 de l'entretoise 10, de diamètre extérieur sensiblement égal au diamètre extérieur du réservoir 2, contre le bord extérieur frontal de l'ouverture du réservoir 2.

L'entretoise 10 porte un embout 11 en forme de doigt de gant. A cet effet, l'entretoise 10 comprend un logement cylindrique axial 13 prolongé selon un passage coaxial 14 de diamètre plus petit, prolongé lui même selon un évasement 15 débouchant à l'extérieur. Au droit de cet évasement 15, l'entretoise porte un filetage extérieur 16 à la surface d'une portion cylindrique d'extrémité de diamètre plus petit que celui de la partie de l'entretoise 10 emmanchée dans le réservoir 2.

A l'extrémité de l'entretoise 10, opposée à celle portant le filetage 16, l'entretoise est munie d'un renflement annulaire 17 de claquage. Dans le logement cylindrique 13 est logé l'embout 11. Celui-ci est en forme d'un doigt de gant définissant une chambre cylindrique 25 et un fond 20. Le fond présente un siège 24 percé d'un orifice capillaire 23 apte à retenir par effet de tension superficielle une certaine quantité de produit 5, sous forme d'une goutte 6. Le fond 20 est bordé d'une collerette 21 de claquage de forme complémentaire de celle du renflement annulaire 17 de l'entretoise 10, permettant ainsi la solidarisation par claquage de l'embout 11 à l'entretoise 10. Le fond de l'embout 11 est à distance du fond 3 du réservoir 2 contenant la composition liquide à mettre au contact de l'applicateur.

L'ensemble d'application comprend également un capuchon 4 représenté partiellement sur les figures 1 et 2. Le capuchon a également la forme d'un cylindre fermé en l'une de ses extrémités, l'autre extrémité étant ouverte. Par cette extrémité ouverte, est emmanchée à force un bouchon 40 de même section que le capuchon 4, qui à lui même une section analogue à celle du réservoir 2. Le bouchon 40 est emmanché dans le capuchon 4 jusqu'à la venue en butée d'une collerette 45 du bouchon 40 contre le bord extérieur de l'ouverture du capuchon 4. Le bouchon 40 présente un chapeau 41 dans lequel est ménagée une cavité 42 prolongée d'un alésage 43 de plus grand diamètre portant intérieurement un filetage 44 complémentaire du filetage 16 porté par l'entretoise 10.

Le bouchon est solidaire d'un porte applicateur 30 supportant un élément d'application 35 (représenté sous forme d'un raisin, par exemple de rouge à lèvres). Typiquement un tel applicateur comprend un produit conditionné sous forme d'un stick solide ou semi solide. Le produit est apte à se solubiliser ou se gélifier en surface au contact d'une composition liquide appropriée, en vue de son délitage sur la peau par exemple. De préférence, il s'agit d'un produit non délitable ou très peu délitable à sec, mais qui le devient en surface quand il est mis au contact d'une composition liquide. Préférentiellement, le produit n'absorbe ou ne pompe pas le liquide en profondeur, de sorte que la solubilisation ou la gélification se produise uniquement en surface. Un tel stick peut être obtenu par moulage ou extrusion. Le porte applicateur 30 comprend une tige creuse 31, percée d'un alésage 32, dont une extrémité porte des ailes longitudinales 33 pour le maintien de la tige 31 dans la cavité 42 du chapeau 41 par emmanchement à force. L'autre extrémité de la tige 31 est munie d'un manchon de maintien 34 rigide, en forme de doigt de gant ouvert, à l'intérieur duquel est monté à force l'applicateur. Dans une variante non représentée, l'applicateur 35 est porté directement par la tige creuse 31.

Selon l'invention, on dispose dans le fond de l'embout 11, un élément élastiquement déformable 100. Cet élément est constitué avantageusement d'un bloc de mousse à cellules ouvertes ou semi ouvertes, ou d'un bloc d'élastomère basse dureté, les cellules ouvertes ou semi ouvertes communiquant entre elles lorsque l'élément déformable n'est sensiblement pas déformé. On entend par élastomère basse dureté, un élastomère dont la dureté est comprise entre 15 shore A et 70 shore A. Cet élastomère est également conformé en mousse à cellules ouvertes ou semi ouvertes. Une face 101 de l'élément déformable 100 est en regard du fond de l'embout 11 et de l'orifice 23 apte à retenir par effet de tension superficielle une quantité de la composition liquide 5. L'autre face 102 est en regard de l'applicateur 35. L'applicateur 35 est monté de sorte que, en position fermée du capuchon 4, le bout de l'applicateur soit en appui sur la surface de restitution 102 de l'élément déformable 100, de manière à le comprimer selon l'axe X de l'ensemble d'application, contre le fond de l'embout 11 et l'orifice 23. Ainsi, l'élément déformable étant chargé en liquide 5, l'applicateur 35 est, dans cette position fermée, en contact dudit liquide. Dans cette position fermée illustrée à la figure 1, le capuchon 4 est vissé sur le réservoir, un joint d'étanchéité 50 étant placé autour de la tige 31, entre le bouchon 40 et l'entretoise 10. D'autres arrangements encore peuvent être utilisés de manière conventionnelle pour assurer l'étanchéité du conditionnement.

La figure 2 représente l'ensemble d'application dans une position ouverte, juste avant utilisation. Dans cette position, l'applicateur 35 n'est plus en appui sur l'élément déformable 100. Le bout de l'applicateur au contact du liquide s'est solubilisé ou gélifié en surface. Le produit peut alors être délité, sur la peau par exemple. L'élément déformable 100 a repris sa forme initiale en pompant une partie au moins de la goutte 6 de produit, le restant 7 du produit, obstruant l'orifice 23 dans lequel il est maintenu par effet de tension superficielle. Ainsi, après utilisation, en revissant le capuchon 4, on remet l'applicateur en contact avec la surface de restitution 102 de l'élément déformable, lequel est imbibé de produit 5. En réalité, il est à noter que, selon l'invention, le transfert de liquide sur l'applicateur se fait lors de sa mise en appui de l'applicateur sur l'élément déformable, mais également de manière simultanée au retrait de l'applicateur, tant que ce dernier est en contact de la mousse. En effet, on peut prévoir, comme on le verra plus en détail par la suite, une valve dont l'ouverture est commandée par le passage en position de repos de l'élément déformable, c'est à dire lors de sa décompression.

Avantageusement, la capacité de pompage de l'élément déformable est supérieure à la quantité de liquide transférée sur l'applicateur, de sorte que l'on peut imbiber ou humecter à nouveau l'applicateur sans avoir à revisser le capuchon sur le réservoir. A cet effet, on prévoit une épaisseur suffisante de mousse pour permettre le pompage souhaité, et on prévoit des fentes ou orifices en nombre et en taille suffisants pour retenir une quantité suffisante de liquide par effet de tension superficielle ou capillarité.

Une bille 60 est logée dans le réservoir 2 et permet l'agitation, si nécessaire du liquide 5. L'orifice 23 débouche dans le réservoir 2 selon une portion évasée tronconique 22. Grâce à cet orifice 23, dont le diamètre peut être de l'ordre de 0,5 mm à 3 mm, une goutte de produit 6 s'accroche par effet de tension superficielle ou capillarité au fond de l'embout 11.

D'autres arrangements encore peuvent être utilisés pour le chargement de l'élément déformable en liquide. A titre d'exemple, on peut citer un organe sous forme d'une mèche (de feutre par exemple) dont une extrémité est au contact de la composition liquide.

L'élément déformable peut être monté sur le fond de l'embout 11 par n'importe quel moyen approprié. A titre d'exemple, il peut être monté par collage, par soudure, par bouterollage, ou par claquage, par l'intermédiaire d'une collerette annulaire.

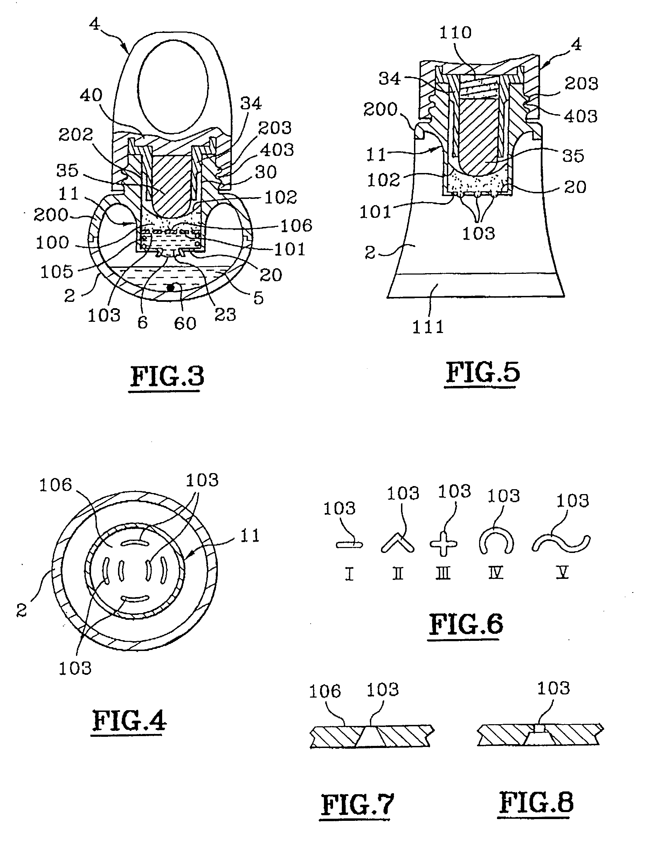

La figure 3 représente une variante de l'ensemble d'application dans laquelle l'élément déformable 100 est monté sur le fond de l'embout 11 par l'intermédiaire d'une plaque 106 montée sur un ressort 105. Lorsque l'applicateur est retiré, la plaque 106 remonte sous l'effet de la force de rappel du mécanisme à ressort 105, en provoquant un effet de piston de manière à favoriser la remontée du produit liquide dans l'élément déformable au travers des orifices ou fentes 103 ménagés dans la plaque 106, comme on le verra plus en détail par la suite. A des fins d'illustration et de clarté, l'espace entre le fond 20 et la plaque 106 a été volontairement exagéré. Le fond de l'embout est lui même percé d'un orifice 23, permettant au liquide de communiquer avec la plaque 106 et apte également à retenir du liquide par effet de tension superficielle. Cet arrangement à ressort permet de compenser le raccourcissement de l'applicateur 35 au fil des utilisations. Ainsi que mentionné précédemment, la plaque 106 est percée d'une ou de plusieurs fentes 103 aptes à retenir du liquide par effet de tension superficielle. Dans l'exemple représenté à la figure 4 (vue en coupe du dispositif de la figure 3 au niveau de la plaque 106), la plaque comporte six fentes en forme d'arcs de cercle. Alternativement, ces fentes sont disposées radialement dans la plaque 106. Bien entendu, ces fentes peuvent avoir, en section transversale, toutes formes appropriées, comme par exemple celles représentées sur les figures 6/I à 6/V. Ces fentes 103 peuvent avoir, en section longitudinale, des formes tronconiques (figure 7) ou partiellement tronconiques (figure 8), de façon à ménager une réserve de produit. Typiquement, de telles fentes ont une section comprise entre 0,2 et 7 mm². Selon une alternative, l'élément déformable est monté sur une grille.

L'embout 11 de cette variante et en un matériau rigide et porte des filets 203 à la surface extérieure de sa partie supérieure 202. Ladite partie supérieure 202 se prolonge vers le bas selon une jupe évasée 200 entourant l'embout 11, en étant à distance radiale de celui-ci. La jupe 200 coiffe le réservoir 2, de forme sensiblement hémisphérique, et est solidarisée au réservoir 2, par exemple par collage ou soudure. Avantageusement, le réservoir est réalisé, au moins en partie en une matière transparente ou translucide, de manière à permettre la visualisation du niveau de liquide dans le réservoir. Les filets 203 de l'embout 11 coopèrent avec des filets 403 du capuchon 4. Dans cette variante, l'applicateur 35 est monté à force par exemple sur un doigt de gant ouvert 34, monté directement de manière étanche sur le col du réservoir 2.

Selon la figure 5, l'ensemble d'application est analogue à celui de la figure 3, sauf que le réservoir 2 est un tube souple raccordé à la jupe évasée 200, ici plus courte que celle de la variante de la figure 3. Le fond du tube est fermé au niveau d'une ligne de soudure transversale 111. Par ailleurs, le raccourcissement de l'applicateur 35 au fil des utilisations est compensé en montant l'applicateur 35 à l'intérieur du doigt de gant ouvert 34 par l'intermédiaire d'un ressort 110, dont la force de rappel pousse l'applicateur en direction du fond du réservoir. Selon cette variante, l'applicateur peut être monté à l'intérieur d'une cupule (non représentée) solidaire du ressort 110.

Ces arrangements à ressorts illustrés en référence aux figures 3 et 5 ne sont nécessaires que dans le cas ou l'applicateur subit un raccourcissement substantiel au fil des utilisations. Lorsque ce raccourcissement est faible (inférieur à 5 ou 7 mm) la compensation du raccourcissement est réalisée par l'élément déformable lui même, dont l'épaisseur et la densité seront choisies de manière adéquate.

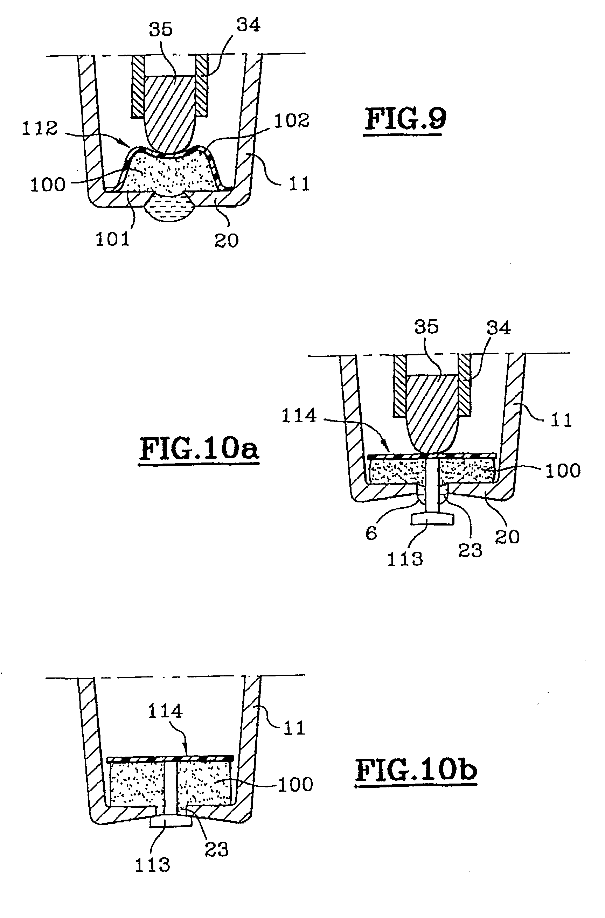

La figure 9 illustre une autre variante de montage de l'élément déformable dans le fond de l'embout 11. Selon cette variante, l'applicateur 35 est au contact de l'élément déformable 100 au travers d'un élément, de consistance souple à rigide ou semi-rigide, perforé ou poreux 112. A titre d'exemple, on utilise une feuille d'élastomère thermoplastique, ou une feuille de plastique. Alternativement, la surface de restitution 102 peut être recouverte d'un revêtement de flocage, ou d'une couche de feutre, ou d'une feuille textile. Ces dispositions permettent de modifier de manière substantielle la capacité de restitution de l'élément déformable vers l'applicateur, ainsi que l'abrasivité de l'élément déformable.

Les figures 10a et 10b illustrent encore une variante de l'invention selon laquelle, l'orifice 23 est obturé de sélectivement au moyen d'une valve 113 dont la tige traverse l'élément déformable 100 et est montée sur un plateau perforé 114 (par exemple en élastomère), sur lequel vient en appui l'applicateur, lorsque le capuchon est vissé sur le réservoir. Dans cette position, illustrée à la figure 10a, l'applicateur appuie sur la plaque perforée, provoquant une descente de la valve 113, ce qui dégage l'orifice 23. En agitant le réservoir, ou en le retournant, par exemple, une quantité de liquide 6 peut s'accrocher sur les parois de l'orifice 23. Lorsque l'on extrait l'applicateur, la valve 113 remonte et obture de manière étanche l'orifice 23, le liquide 6 étant alors absorbé par pompage dans l'élément déformable 100 qui reprend son volume initial. Cette configuration permet de fermer de manière étanche le réservoir lorsque celui-ci n'est pas fermé par le capuchon, évitant ainsi tout risque de souillure intempestive.

Selon une alternative, non représentée, la valve 113 est montée de telle sorte quelle obture l'orifice 23 lorsque l'applicateur est en appui sur l'élément déformable, et qu'elle le dégage lorsque l'on retire l'applicateur 35. A cet effet, et à titre d'exemple, le clapet de la valve 113 est à l'intérieur du doigt de gant 11, la mise en appui de l'applicateur 35 sur l'élément déformable 100 provoquant la mise en appui de manière étanche du clapet sur l'orifice. L'élasticité du matériau formant la mousse 100 est suffisante pour rappeler la valve en position ouverte lorsque l'on extrait l'applicateur. Avantageusement, la valve est montée sur un ressort auxiliaire (non représenté) permettant l'ouverture de la valve lors du retrait de l'applicateur. Cette configuration permet de limiter les risques de pompage excessif du produit formant l'applicateur, et ainsi, de imiter les risques d'une solubilisation ou d'une gélification en profondeur du produit. Cette caractéristique est particulièrement intéressante pour certains produits dont le processus de solubilisation ou de gélification s'intensifie avec le temps de mise en contact avec la composition liquide, et/ou avec le volume déjà solubilisé ou gélifié.

L'invention qui vient d'être décrite est particulièrement avantageuse, en ce qu'elle permet de manière simple et efficace de maintenir avant application, la surface d'un applicateur en contact avec une composition liquide donnée. Le réglage de la quantité de liquide délivrée sur l'applicateur pourra se faire de diverses manières: soit en ajustant le niveau de pression de l'applicateur sur la surface de restitution, soit en choisissant de manière appropriée la densité et l'épaisseur de l'élément déformable, soit en modifiant la surface de restitution de la mousse, par flocage par exemple, ou en intercalant entre l'élément déformable et l'applicateur une feuille d'un matériau apte à modifier de manière sensible la capacité de restitution de la mousse. Il est possible par ailleurs de jouer sur la taille, le nombre et la forme des orifices au contact de la surface de chargement de l'élément déformable, de manière à limiter l'absorption liquide par ledit élément déformable.

Dans la description qui précède, il a été fait référence à des modes de réalisation préférés de l'invention. Il est évident que des variantes peuvent y être apportées sans s'écarter de l'esprit de l'invention telle que revendiquée ci-après.

## Revendications

1. Ensemble de conditionnement et d'application (1) d'axe principal (X), comportant un applicateur (35), et un réservoir (2) contenant une composition liquide (5) au contact de laquelle doit être mis l'applicateur (35) avant application, caractérisé en ce que l'applicateur contient un produit apte à se solubiliser ou se gélifier en surface au contact de ladite composition liquide, l'applicateur étant mis en contact avec la composition liquide au travers d'un élément élastiquement déformable (100) apte à contenir une quantité de ladite composition liquide, et pouvant passer sélectivement d'une position comprimée dans laquelle au moins une partie de la surface de l'applicateur est en appui, selon l'axe principal (X), sur une surface de restitution (102) de l'élément déformable de manière à être en contact avec la composition liquide, à une position de repos, le passage de la position comprimée à la position de repos provoquant le chargement de l'élément déformable (100) en composition liquide par pompage au travers d'une surface de chargement (101) de l'élément déformable.

2. Ensemble de conditionnement et d'application selon la revendication 1 caractérisé en ce que l'élément élastiquement déformable (100) est en mousse ou en élastomère basse dureté, et est porté par le réservoir (2), un capuchon (4) étant monté de manière amovible sur le réservoir et portant un porte applicateur (31, 34) sur lequel est monté l'applicateur (35), ledit applicateur étant, en position fermée du capuchon en appui sur la surface de restitution (102) de l'élément déformable de manière à comprimer ledit élément déformable (100) selon l'axe principal (X) et à maintenir l'applicateur (35) au contact avec la composition liquide, l'ouverture du capuchon (4) et la sortie de l'applicateur (35) provoquant par effet de pompage le chargement de l'élément déformable au travers de la surface de chargement (101).

3. Ensemble de conditionnement et d'application selon la revendication 2 caractérisé en ce que l'élément déformable est monté à l'intérieur d'un organe (11) de type doigt de gant comportant un fond au contact duquel est placé la surface de chargement, ledit fond présentant des moyens (23, 103) aptes à retenir par effet de tension superficielle ou capillarité une quantité (6) de ladite composition liquide.

4. Ensemble de conditionnement et d'application selon la revendication 3 caractérisé en ce que le fond du doigt de gant est constitué d'une grille.

5. Ensemble de conditionnement et d'application selon la revendication 3 caractérisé en ce que lesdits moyens (23, 103) comportent au moins un orifice (23) et/ou au moins une fente (103), ménagés dans le fond (20) du doigt de gant (11).

6. Ensemble de conditionnement et d'application selon la revendication 5 caractérisé en ce que le fond (20) du doigt de gant comporte une pluralité de fentes (103) formant des portions concentriques d'arcs de cercles.

7. Ensemble de conditionnement et d'application selon la revendication 5 caractérisé en ce que le fond du doigt de gant comporte une pluralité de fentes radiales (103).

8. Ensemble de conditionnement et d'application selon l'une quelconque des revendications 5 à 7 caractérisé en ce que les fentes et/ou les orifices (23, 103) débouchent dans le réservoir selon une portion évasée, tronconique, et faisant office de réservoir par effet de tension superficielle ou par effet de capillarité.

9. Ensemble de conditionnement et d'application selon la revendication 5, caractérisé en ce que le fond comporte un orifice central, pouvant être obturé sélectivement par une valve (113) dont l'ouverture est provoquée par la compression de l'élément déformable (100), l'ouverture du capuchon (4) et la sortie de l'applicateur (35) provoquant la fermeture de la valve (113).

10. Ensemble de conditionnement et d'application selon la revendication 5, caractérisé en ce que le fond comporte un orifice central, pouvant être obturé sélectivement par une valve (113) dont la fermeture est provoquée par la compression de l'élément déformable (100), l'ouverture du capuchon (4) et la sortie de l'applicateur (35) provoquant l'ouverture de la valve (113).

11. Ensemble de conditionnement et d'application selon l'une quelconque des revendications 2 à 10 caractérisé en ce que l'applicateur (35) est formé d'un raisin d'un produit solide ou semi solide, délitable en surface au contact d'une composition liquide, et est monté sur le porte applicateur par l'intermédiaire d'un mécanisme formant ressort (110) de manière à compenser le raccourcissement de l'applicateur (35) au fil des utilisations.

12. Ensemble de conditionnement et d'application selon l'une quelconque des revendications 2 à 11 caractérisé en ce que l'élément déformable (100) est monté dans le fond du doigt de gant par l'intermédiaire d'un mécanisme formant ressort (105).

13. Ensemble de conditionnement et d'application selon l'une quelconque des revendications 3 à 12 caractérisé en ce que l'élément déformable (100) est monté dans le fond du doigt de gant (11) par collage, soudure, bouterollage, ou par claquage par l'intermédiaire d'une collerette annulaire.

14. Ensemble de conditionnement et d'application selon la revendication 1 à 13 caractérisé en ce que l'élément déformable (100) est constitué d'une mousse ou d'un élastomère, à cellules ouvertes ou semi ouvertes, dont la surface de restitution est recouverte d'un revêtement de flocage, ou d'un film perforé d'élastomère thermoplastique (112), de plastique, ou d'une couche de feutre, ou d'un textile.

15. Ensemble de conditionnement et d'application selon l'une quelconque des revendications 1 à 14 caractérisé en ce que le réservoir (2) est constitué d'un tube.

16. Ensemble de conditionnement et d'application selon l'une quelconque des revendications 1 à 15 caractérisé en ce que le réservoir (2) est au moins en partie constitué d'un matériau translucide de manière à permettre de visualiser la quantité de composition liquide restant dans le réservoir.

17. Ensemble de conditionnement et d'application selon l'une quelconque des revendications précédentes caractérisé en ce que l'applicateur (35) est une pierre d'alun, un raisin de rouge à lèvres, d'alcool polyvinylique (PVA), de gomme de guar, ou d'un produit de traitement cutané.

18. Ensemble de conditionnement et d'application selon l'une quelconque des revendications 1 à 17 caractérisé en ce que la composition liquide (5) contient de l'eau ou tout autre solvant, et/ou des conservateurs, et/ou des émollients et/ou des composés actifs.

## Claims

1. Storage case and applicator assembly (1) with principal axis (X), which includes an applicator (35) and a reservoir (2) containing a liquid composition (5) with which the applicator (35) must be placed in contact before application, characterized in that the applicator contains a product capable of becoming soluble or assuming a gel consistency on the surface in contact with the said liquid composition, the applicator being placed in contact with the liquid composition by means of an elastically deformable element (100) capable of containing a quantity of the said liquid composition and being able to move selectively from a compressed position, in which at least one portion of the surface of the applicator bears, along the principal axis (X), on a restoration surface (102) of the deformable element so as to be in contact with the liquid composition, to a rest position, the transition from the compressed position to the rest position giving rise to the loading of the deformable element (100) with liquid composition by means of pumping via a loading surface (101) of the deformable element.

2. Storage case and applicator assembly according to Claim 1, characterized in that the elastically deformable element (100) is made from foam or from low-hardness elastomer and is carried by the reservoir (2), a cap (4) being mounted removably on the reservoir and carrying an applicator-holder (31, 34), on which the applicator (35) is mounted, the said applicator, in the closed position of the cap, bearing on the restoration surface (102) of the deformable element so as to compress the said deformable element (100) along the principal axis (X) and to keep the applicator (35) in contact with the liquid composition, opening of the cap (4) and removal of the applicator (35) giving rise, through a pumping effect, to the loading of the deformable element via the loading surface (101).

3. Storage case and applicator assembly according to Claim 2, characterized in that the deformable element is mounted inside a member (11) of the glove-finger type, which includes a base with which the loading surface is placed in contact, the said base having means (23, 103) capable of retaining a quantity (6) of the said liquid composition by surface-tension or capillary effect.

4. Storage case and applicator assembly according to Claim 3, characterized in that the base of the glove finger consists of a grid.

5. Storage case and applicator assembly according to Claim 3, characterized in that the said means (23, 103) include at least one orifice (23) and/or at least one slot (103) which are made in the base (20) of the glove finger (11).

6. Storage case and applicator assembly according to Claim 5, characterized in that the base (20) of the glove finger includes a plurality of slots (103) forming concentric portions of arcs of circles.

7. Storage case and applicator assembly according to Claim 5, characterized in that the base of the glove finger includes a plurality of radial slots (103).

8. Storage case and applicator assembly according to any one of Claims 5 to 7, characterized in that the slots and/or the orifices (23, 103) emerge in the reservoir in a flared, frustoconical portion acting as a reservoir by surface-tension effect or capillary effect.

9. Storage case and applicator assembly according to Claim 5, characterized in that the base includes a central orifice which may be closed off selectively by a valve (113) which is opened by the compression of the deformable element (100), opening of the cap (4) and removal of the applicator (35) giving rise to closure of the valve (113).

10. Storage case and applicator assembly according to Claim 5, characterized in that the base includes a central orifice which may be closed off selectively by a valve (113) which is closed by the compression of the deformable element (100), opening of the cap (4) and removal of the applicator (35) giving rise to the opening of the valve (113).

11. Storage case and applicator assembly according to any one of Claims 2 to 10, characterized in that the applicator (35) is formed from a grape-sized stick of a solid or semi-solid product which can be flaked at the surface in contact with a liquid composition and is mounted on the applicator-holder by means of a mechanism which forms a spring (110) so as to offset the shortening of the applicator (35) as use progresses.

12. Storage case and applicator assembly according to any one of Claims 2 to 11, characterized in that the deformable element (100) is mounted in the base of the glove finger by means of a mechanism which forms a spring (105).

13. Storage case and applicator assembly according to any one of Claims 3 to 12, characterized in that the deformable element (100) is mounted in the base of the glove finger (11) by bonding, welding, riveting or by snap-fitting by means of an annular collar.

14. Storage case and applicator assembly according to Claims 1 to 13, characterized in that the deformable element (100) consists of a foam or of an elastomer, with open or semi-open cells, the restoration surface of which is covered with a flock covering or with a perforated plastic or thermoplastic elastomer film (112), or with a layer of felt or with a textile.

15. Storage case and applicator assembly according to any one of Claims 1 to 14, characterized in that the reservoir (2) consists of a tube.

16. Storage case and applicator assembly according to any one of Claims 1 to 15, characterized in that the reservoir (2) consists at least partially of a translucent material so as to enable the quantity of liquid composition remaining in the reservoir to be seen.

17. Storage case and applicator assembly according to any one of the preceding claims, characterized in that the applicator (35) is a piece of alum, a grape-sized stick of lipstick, polyvinyl alchohol (PVA), guar gum or a skin-treatment product.

18. Storage case and applicator assembly according to any one of Claims 1 to 17, characterized in that the liquid composition (5) contains water or any other solvent and/or preservatives and/or emollients and/or active compounds.

## Patentansprüche

1. Einheit (1) zur Verpackung und zum Auftragen mit einer Hauptachse (X), die einen Applikator (35) und einen eine flüssige Zusammensetzung (5) enthaltenden Behälter (2) aufweist, mit der der Applikator (35) vor dem Auftragen in Kontakt gebracht werden muß, dadurch gekennzeichnet, daß der Applikator ein Produkt enthält, das sich in Kontakt mit der flüssigen Zusammensetzung an der Oberfläche lösen oder Gelform annehmen kann, wobei der Applikator durch ein elastisch verformbares Element (100) hindurch mit der flüssigen Zusammensetzung in Kontakt gebracht wird, das eine Menge der flüssigen Zusammensetzung enthält und wahlweise von einer komprimierten Stellung, in der mindestens ein Teil der Oberfläche des Applikators gemäß der Hauptachse (X) auf einer Wiedergabefläche (102) des verformbaren Elements aufliegt, um mit der flüssigen Zusammensetzung in Kontakt zu sein, in eine Ruhestellung übergehen kann, wobei der Übergang von der komprimierten Stellung in die Ruhestellung das Laden des verformbaren Elements (100) mit flüssiger Zusammensetzung mittels Pumpen durch eine Ladefläche (101) des verformbaren Elements hindurch hervorruft.

2. Einheit zur Verpackung und zum Auftragen gemaß Anspruch 1, dadurch gekennzeichnet, daß das elastisch verformbare Element (100) aus Schaumstoff oder einem Elastomermaterial geringer Härte besteht und auf dem Behälter (2) sitzt, wobei eine Verschlußkappe (4) lösbar auf dem Behälter angebracht ist und einen Applikator-Träger (31, 34) trägt, auf den der Applikator (35) montiert ist, wobei der Applikator in der geschlossenen Stellung der Verschlußkappe auf der Wiedergabefläche (102) des verformbaren Elements aufliegt, so daß das verformbare Element (100) gemäß der Hauptachse (X) komprimiert und der Applikator (35) mit der flüssigen Zusammensetzung in Kontakt gehalten wird, wobei die Öffnung der Verschlußkappe (4) und der Austritt des Applikators (35) durch Pumpwirkung das Laden des verformbaren Elements durch die Ladefläche (101) hindurch bewirken.

3. Einheit zur Verpackung und zum Auftragen gemäß Anspruch 2, dadurch gekennzeichnet, daß das verformbare Element innerhalb eines Organs (11) der Art Handschuhfinger angeordnet ist, das einen Boden besitzt, in Kontakt mit dem die Ladefläche angeordnet ist, wobei der Boden Mittel (23, 103) aufweist, die durch Oberflächenspannung oder Kapillarwirkung eine Menge (6) der flüssigen Zusammensetzung zurückhalten können.

4. Einheit zur Verpackung und zum Auftragen gemäß Anspruch 3, dadurch gekennzeichnet, daß der Boden des Handschuhfingers aus einem Gitter besteht.

5. Einheit zur Verpackung und zum Auftragen gemäß Anspruch 3, dadurch gekennzeichnet, daß die Mittel (23, 103) mindestens eine Öffnung (23) und/oder mindestens einen Schlitz (103) aufweisen, die im Boden (20) des Handschuhfingers (11) ausgearbeitet sind.

6. Einheit zur Verpackung und zum Auftragen gemäß Anspruch 5, dadurch gekennzeichnet, daß der Boden (20) des Handschuhfingers eine Vielzahl von Schlitzen (103) aufweist, die konzentrische Abschnitte von Kreisbögen bilden.

7. Einheit zur Verpackung und zum Auftragen gemäß Anspruch 5, dadurch gekennzeichnet, daß der Boden des Handschuhfingers eine Vielzahl von radialen Schlitzen (103) aufweist.

8. Einheit zur Verpackung und zum Auftragen gemäß einem beliebigen der Ansprüche 5 bis 7, dadurch gekennzeichnet, daß die Schlitze und/oder Öffnungen (23, 103) in den Behälter gemäß einem kegelstumpfförmig ausgeweiteten Abschnitt münden, der durch Oberflächenspannung oder Kapillarwirkung als Behälter dient.

9. Einheit zur Verpackung und zum Auftragen gemäß Anspruch 5, dadurch gekennzeichnet, daß der Boden eine zentrale Öffnung aufweist, die wahlweise von einem Ventil (113) verschlossen werden kann, dessen Öffnung durch die Komprimierung des verformbaren Elements (100) hervorgerufen wird, während die Öffnung der Verschlußkappe (4) und das Austreten des Applikators (35) das Schließen des Ventils (113) verursachen.

10. Einheit zur Verpackung und zum Auftragen gemäß Anspruch 5, dadurch gekennzeichnet, daß der Boden eine zentrale Öffnung aufweist, die wahlweise von einem Ventil (113) verschlossen werden kann, dessen Schließen durch die Komprimierung des verformbaren Elements (100) hervorgerufen wird, während die Öffnung der Verschlußkappe (4) und das Austreten des Applikators (35) die Öffnung des Ventils (113) verursachen.

11. Einheit zur Verpackung und zum Auftragen gemäß einem beliebigen der Ansprüche 2 bis 10, dadurch gekennzeichnet, daß der Applikator (35) die Form einer Traube aus einem festen oder halbfesten Produkt hat, das an der Oberfläche in Kontakt mit einer flüssigen Zusammensetzung ablösbar ist, und auf den Applikator-Träger mittels eines federförmigen Mechanismus (110) montiert ist, um die Verkürzung des Applikators (35) im Lauf der Anwendungen zu kompensieren.

12. Einheit zur Verpackung und zum Auftragen gemäß einem beliebigen der Ansprüche 2 bis 11, dadurch gekennzeichnet, daß das verformbare Element (100) mittels eines als Feder wirkenden Mechanismus (105) am Boden des Handschuhfingers montiert wird.

13. Einheit zur Verpackung und zum Auftragen gemäß einem beliebigen der Ansprüche 3 bis 12, dadurch gekennzeichnet, daß das verformbare Element (100) am Boden des Handschuhfingers (11) durch Kleben, Schweißen, Vernieten oder Einschnappen mit Hilfe eines ringförmigen Kragens angeordnet wird.

14. Einheit zur Verpackung und zum Auftragen gemäß den Ansprüchen 1 bis 13, dadurch gekennzeichnet, daß das verformbare Element (100) aus einem Schaumstoff oder einem Elastomermaterial mit offenen oder halboffenen Zellen besteht, dessen Wiedergabefläche mit einem Beflockungs-Belag oder einer gelochten Folie aus thermoplastischem Elastomermaterial (112), aus Kunststoff oder einer Filz- oder Textilschicht bedeckt ist.

15. Einheit zur Verpackung und zum Auftragen gemäß einem beliebigen der Ansprüche 1 bis 14, dadurch gekennzeichnet, daß der Behälter (2) aus einer Tube besteht.

16. Einheit zur Verpackung und zum Auftragen gemäß einem beliebigen der Ansprüche 1 bis 15, dadurch gekennzeichnet, daß der Behälter (2) zumindest teilweise aus einem durchscheinenden Material besteht, um die Sichtbarmachung der Menge an im Behälter verbleibender flüssiger Zusammensetzung zu ermöglichen.

17. Einheit zur Verpackung und zum Auftragen gemäß einem beliebigen der vorhergehenden Ansprüche, dadurch gekennzeichnet, daß der Applikator (35) ein Alaunstein, eine Lippenstift-Traube, ein Stift aus Polyvinylalkohol (PVA), aus Guargummi oder aus einem Produkt zur Hautbehandlung ist.

18. Einheit zur Verpackung und zum Auftragen gemäß einem beliebigen der Ansprüche 1 bis 17, dadurch gekennzeichnet, daß die flüssige Zusammensetzung (5) Wasser oder ein beliebiges anderes Lösungsmittel und/oder Konservierungsstoffe und/oder Weichmacher und/oder aktive Verbundstoffe enthält.
